# EUROPÄISCHE PATENTSCHRIFT

(11) **EP 2 767 294 B1**
(45) Veröffentlichungstag und Bekanntmachung des Hinweises auf die Patenterteilung: **21.03.2018**
(21) Anmeldenummer: 13193265.9
(22) Anmeldetag: 18.11.2013
(51) Int. Cl.: A61L 31/02, A61L 31/10, A61L 31/14, A61L 31/16, A61L 31/12

(54) **Biokorrodierbares Implantat mit korrosionshemmender Beschichtung**
Biocorrodible implant with anti-corrosion coating
Implant biocorrodable doté d'un revêtement anticorrosion

(30) Priorität: 13.02.2013 US 201361763956 P
(43) Veröffentlichungstag der Anmeldung: 20.08.2014
(73) Patentinhaber: Biotronik AG, 8180 Bülach (CH)
(72) Erfinder: Wittchow, Eric, 91710 Gunzenhausen (DE)
(74) Vertreter: Randoll, Sören

(56) Entgegenhaltungen:
- EP-A2- 2 415 489
- WO-A2-2009/158325
- US-A1- 2005 261 760
- US-A1- 2009 287 301
- BORDES P ET AL: "Nano-biocomposites: Biodegradable polyester/nanoclay systems", PROGRESS IN POLYMER SCIENCE, PERGAMON PRESS, OXFORD, GB, Bd. 34, Nr. 2, 1. Februar 2009 (2009-02-01), Seiten 125-155, XP025770088, ISSN: 0079-6700, DOI: 10.1016/J.PROGPOLYMSCI.2008.10.002 [gefunden am 2009-01-12]

## Beschreibung

Die Erfindung betrifft ein biokorrodierbares Implantat auf Basis von Magnesium oder einer biokorrodierbaren Magnesiumlegierung, dessen Oberfläche eine korrosionshemmende Beschichtung aufweist.

Unter Implantat wird allgemein jede medizinische Vorrichtung aus einem oder mehreren Werkstoffen verstanden, die absichtlich in den Körper eingebracht wird und entweder teilweise oder ganz von einer Epitheloberfläche bedeckt ist. Implantate lassen sich hinsichtlich der Verwendungsdauer in Temporär- und Permanentimplantate untergliedern. Temporärimplantate verbleiben für einen befristeten Zeitraum im Körper. Permanentimplantate sind für den dauerhaften Verbleib im Körper vorgesehen. Bei Implantaten kann ferner zwischen Prothesen und künstlichen Organe unterschieden werden. Eine Prothese ist eine medizinische Vorrichtung, die Gliedmaßen, Organe oder Gewebe des Körpers ersetzt, während unter einem künstlichen Organ eine medizinische Vorrichtung verstanden wird, die teilweise oder ganz die Funktion eines Körperorgans ersetzt. Unter die genannten Definitionen fallen beispielsweise Implantate wie orthopädische oder osteosynthetische Implantate, Herzschrittmacher und Defibrillatoren und vaskuläre Implantate.

Insbesondere die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die vorrangig zur Abdichtung des Aneuryrismas dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Das Implantat, insbesondere der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise.

Entsprechend der Reaktion des Biosystems können die Implantatswerkstoffe in bioaktive, bioinerte und degradierbare/resorbierbare Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiA16Nb7) und Goldlegierungen. Im Bereich biokorrodierbarer Implantate, insbesondere Stents, wird der Einsatz von Magnesium oder Reineisen sowie biokorrodierbaren Basislegierungen der Elemente Magnesium, Eisen, Zink, Molybdän und Wolfram vorgeschlagen.

Für die Zwecke der vorliegenden Erfindung sind lediglich metallische Implantatwerkstoffe von Interesse, die ganz oder in Teilen aus Magnesium oder biokorrodierbaren Magnesiumlegierungen bestehen (magnesiumhaltige Legierungen).

Eine Limitierung des Einsatzes biokorrodierbarer Magnesiumlegierungen ist die rasche Degradation des Materials in physiologischer Umgebung. Sowohl die Grundlagen der Magnesiumkorrosion als auch eine große Anzahl von technischen Verfahren zur Verbesserung des Korrosionsverhaltens (im Sinne einer Verstärkung des Korrosionsschutzes) sind aus dem Stand der Technik bekannt. Bekannt ist beispielsweise, dass der Zusatz von Yttrium und/oder weiteren Seltenerdmetallen einer Magnesiumlegierungen einen leicht erhöhten Korrosionswiderstand in Meerwasser verleiht.

Ein Ansatzpunkt zur Verbesserung des Korrosionsverhaltens sieht vor, eine korrosionsschützende Schicht auf dem aus Magnesium oder einer Magnesiumlegierung bestehenden Formkörper zu erzeugen. Bekannte Verfahren zur Erzeugung einer korrosionsschützenden Schicht wurden bisher aus dem Gesichtspunkt eines technischen Einsatzes des Formkörpers - jedoch nicht medizintechnischen Einsatzes in biokorrodierbaren Implantaten in physiologischer Umgebung - entwickelt und optimiert. Diese bekannten Verfahren umfassen: das Aufbringen von Polymeren oder anorganischen Deckschichten, das Erzeugen einer Emaille, die chemische Konversion der Oberfläche, Heißgasoxidation, Anodisierung, Plasmaspritzen, Laserstrahl-Umschmelzen, PVD-Verfahren, Ionenimplantation oder Lackieren.

Herkömmliche technische Einsatzgebiete von Formkörpern aus Magnesiumlegierungen außerhalb der Medizintechnik erfordern in der Regel eine weitgehende Unterbindung korrosiver Prozesse. Dementsprechend ist die Zielstellung der meisten technischen Verfahren eine vollständige Eliminierung korrosiver Prozesse. Dagegen sollte die Zielstellung zur Verbesserung des Korrosionsverhaltens von biokorrodierbaren Magnesiumlegierungen nicht in der vollständigen Unterbindung, sondern nur in der Hemmung korrosiver Prozesse liegen. Schon aus diesem Grunde eignen sich die meisten bekannten Verfahren zur Erzeugung von Korrosionsschutzschichten auf Magnesium nicht. Ferner müssen für einen medizintechnischen Einsatz auch toxikologische Aspekte berücksichtigt werden. Des Weiteren sind korrosive Prozesse stark von dem Medium abhängig, in dem sie ablaufen, und daher dürfte eine Übertragbarkeit der unter herkömmlichen Umweltbedingungen auf technischem Gebiet gewonnenen Erkenntnisse zum Korrosionsschutz auf die Prozesse in physiologischer Umgebung nicht uneingeschränkt möglich sein. Schließlich dürfen bei einer Vielzahl von medizinischen Implantaten auch die der Korrosion zugrunde liegenden Mechanismen von üblichen technischen Anwendungen des Werkstoffs abweichen. So werden beispielsweise Stents, chirurgisches Material oder Clips im Gebrauch mechanisch verformt, sodass der Teilprozess der Spannungsrisskorrosion beim Abbau dieser Formkörper erhebliche Bedeutung haben dürfte.

Der Grundkörper einiger Implantate, wie insbesondere von Stents, wird im Gebrauch lokal unterschiedlich starken plastischen Verformungen unterworfen. Konventionelle Verfahren zur Hemmung der Korrosion, wie beispielsweise die Generierung einer dichten Magnesiumoxid-Deckschicht, sind hier nicht zielführend. Die keramischen Eigenschaften einer solchen Deckschicht würden zu einem lokalen Abplatzen oder zumindest Rissbildung der Deckschicht führen. Die Korrosion fände damit in unkontrollierter Weise statt und es bestünde insbesondere die Gefahr, dass in den mechanisch besonders beanspruchten Bereichen des Implantats die Korrosion forciert ist.

Der Auftrag nicht degradierbarerer, polymerer Passivierungsschichten kann zwar den Abbau des Implantats hemmen, jedoch konterkariert er die Grundidee eines völlig degradierbaren Implantats, denn im Körper verbleibt das polymere Material der Passivierungsschicht. Eine vielversprechende Alternative ist daher die Verwendung von biodegradierbaren Polymeren als Werkstoff für eine Passivierungsschicht für Implantate auf Basis von biodegradierbaren Magnesiumwerkstoffen.

Aus EP 2415489 A2, US 2009-0240323 A1, US 2010-0076544 A1 und US 2011/0076319 A1 ist es bekannt, einen Magnesium-Stent mit einer biodegradierbaren polymeren Beschichtung als Passivierungsschicht zu beschichten. Hierdurch soll der Abbau des Grundkörpers aus Magnesium beziehungsweise einer Magnesiumlegierung verlangsamt werden. Als biodegradierbare polymere Beschichtungsmaterialien kommen beispielsweise Polylactide, Polyhydroxyalkanoate, Polycaprolactone, aliphatische Polyester, aromatische Copolyester und Polyesteramide in Frage. Trotz dieser vielversprechenden Ansätze, ist es bisher nicht zufriedenstellend gelungen, die Degradation des metallischen Grundkörpers des Stents hinreichend zu verzögern.

Eines oder mehrere der geschilderten Probleme des Standes der Technik lassen mit Hilfe des erfindungsgemäßen Implantats beheben oder zumindest zu mindern. Das Implantat, vorzugsweise ein Stent, besitzt einen Grundkörper aus Magnesium oder einer biokorrodierbaren Magnesiumlegierung und eine den Grundkörper bedeckende korrosionshemmende Passivierungsschicht. Die Passivierungsschicht zeichnet sich dadurch aus, dass sie ein Komposit aus einem biodegradierbaren Polymer und Nanopartikeln aus organomodifizierten Tonmineralien enthält.

Der Erfindung liegt die Erkenntnis zugrunde, dass mithilfe eine Komposits aus biodegradierbaren Polymeren und Nanopartikel aus organomodifizierten Tonmineralien die Wasserdurchlässigkeit der Passivierungsschicht ganz erheblich gemindert werden kann. Es hat sich in einigen Versuchen herausgestellt, dass gerade eine geringe Wasserdurchlässigkeit ein entscheidender Faktor für die hinreichende Funktionalität der Passivierungsschicht ist. Der Degradationsschutz hängt also im Wesentlichen von der Wasserdurchlässigkeit ab, welche durch die Diffusionskoeffizienten und Schichtdicke bestimmt wird. Durch die Beimischung der Tonmineralien soll die Wasserdurchlässigkeit der Passivierungsschicht insbesondere unter einen Wert der normalisierten Wasserdampfdurchlässigkeit (WVTR) von 50g/m² und Tag gesenkt werden.

Unter dem Begriff ,Tonminerale' werden vorliegend Minerale verstanden, die überwiegend feinstkörnig (Korngröße ≤ 2 µm) vorkommen und Schichtsilikate sind. Tonminerale bestehen aus zwei charakteristischen Bauelementen, einer Tetraederschicht (eckenverknüpfte SiO₄-Tetraeder, zum Teil Si substituiert durch Al) und einer Oktaederschicht (kantenverknüpfte AlO₆-Oktaeder, zum Teil Al substituiert durch Mg).

Je nach Anordnung dieser Schichten unterscheidet man 1:1-Tonminerale (Zweischicht-Tonminerale, zum Beispiel Kaolinit oder Chrysotil), 2:1-Tonminerale (Dreischicht-Tonminerale, zum Beispiel Illit) und 2:1:1-Tonminerale (Vierschicht-Tonminerale, zum Beispiel Chlorit). Durch die Substitution (vor allem von vierwertigem Si durch dreiwertiges Al in der Tetraederschicht oder von dreiwertigem Al durch zweiwertiges Mg in der Oktaederschicht) entsteht eine negative Schichtladung, die durch die Einlagerung von Kationen in der Zwischenschicht neutralisiert wird. Die Schichtladung der 1:1-Tonminerale ist stets Null. Die 2:1-Tonminerale werden nach ihrer Schichtladung weiter unterteilt in Talk-Pyrophyllit-Gruppe, Smektitgruppe (zum Beispiel Montmorillonit, Beidellit, Nontronit, Saponit oder Hectorit), Vermiculit-Illit-Gruppe und Glimmergruppe. Tonminerale mit nicht ganzzahligen Schichtladungen besitzen die Fähigkeit zur Quellung, das heißt zur temporären und reversiblen Wasseraufnahme in ihren Zwischenschichten. Alternativ kann die Schichtladung in der Oktaederschicht auch dadurch kompensiert werden, dass nur zwei von drei Oktaedern besetzt sind (dioktaedrische Tonminerale, zum Beispiel Kaolinit; trioktaedrische Tonminerale, zum Beispiel Chrysotil). Das Tonmineral ist vorzugsweise Montmorillonit oder Saponit.

Zum Einsatz als Füllstoff in der Passivierungsschicht aus dem biodegradierbaren Polymer werden die Tonmineralien zuvor modifiziert, um sie lipophil zu machen.

Dadurch verlieren sie ihre hohe Wasseraufnahmefähigkeit, lassen sich aber mit den biodegradierbaren Polymeren mischen. Die Modifikation kann durch Ersetzen von anorganischen Ionen im Mineral mit substituieren Ammonium- oder Phosphoniumionen erfolgen (organomodifizierte Tonminerale; siehe Perrine Bordes, Eric Pollet, Luc Averous, Nano-biocomposites: Biodegradable polyester/nanoclay systems, Progress in Polymer Science 34 (2009), 125 - 155). Das organomodifizierte Tonmineral ist vorzugsweise Montmorillonit oder Saponit. Die als Füllstoff verwendeten Tonminerale weisen in der Regel Schichtdicken von etwa 1 nm und Schichtlängen von bis zu 2 µm auf.

Die Verbindung von polymerer Matrix und Tonmineral kann auf 3 Arten erreicht werden:
a) Solvent Intercalation Route, bei der das Mineral in einer polymerhaltigen Lösung aufgeschwemmt wird, wodurch das Makromolekül in die Hohlräume der Tonerde diffundiert
b) In-situ Intercalation Methode, in der das Mineral in einer monomerhaltigen Lösung aufgeschwemmt wird, ehe diese polymerisiert wird
c) Melt Intercalation Process, bei der die Vermischung im geschmolzenen Zustand des Polymers stattfindet, wie z.B. bei der Extrusion

Ferner ist bevorzugt, wenn das biodegradierbare Polymer ausgewählt ist aus der Gruppe umfassend Poly(lactide), Polyhydroxyalkonate, Polycaprolactone, aliphatische Polyester, aromatische Copolyester und Polyesteramide. Besonders bevorzugt ist das biodegradierbare Polymer Polyhydroxyalkonat. Die genannten biodegradierbaren Polymere lassen sich besonders einfach zu den gewünschten Kompositen umsetzen und besitzen in der Regel das für die erfindungsgemäßen Zwecke geeignete Degradationsverhalten.

Die Passivierungsschicht kann weitere Hilfsstoffe und vorzugsweise auch Wirkstoff enthalten, die nach der Implantation freigesetzt werden.

Der pharmakologische Wirkstoff ist bevorzugt ausgewählt aus der Gruppe umfassend Limus-Verbindungen, bevorzugt Sirolimus (Rapamycin), und Cytostatika, vorzugsweise Paclitaxel.

Unter Magnesiumlegierung wird vorliegend ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C und pH 7,38 gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Vorzugsweise ist das Implantat ein Stent.

### Ausführungsbeispiel:

Das Nanokomposit wird durch die solution intercalation Methode (Lösung-Einlagerungs-Methode) hergestellt. Organomodifiziertes Montmorillonit (OMMT) wird als Pulver zu einer Lösung aus PHBV (Poly(3-hydroxybutyrat-co-3-hydroxyvalerat)) in Chloroform zugegeben, um einen Gewichtanteil von 5 Gew% von OMMT zu erhalten (Herstellung erfolgt analog zu Chen et al., Journal of Material Science Letter 21 (2002), 1587 - 1589).

Die Dispersion wird im Ultraschallbad für weitere 3 Stunden dispergiert und vor der Weiterverarbeitung für 1h auf 60°C Grad unter Rückfluss erhitzt. Nach Abkühlen der Lösung auf Raumtemperatur wird die Lösung in einem Sprayverfahren auf die Oberfläche eines Stents aufgebracht, der aus einer biokorrodierbaren Magnesiumlegierung geformt ist.

## Patentansprüche

1. Implantat mit einem Grundkörper aus Magnesium oder einer biokorrodierbaren Magnesiumlegierung und einer den Grundkörper bedeckenden korrosionshemmenden Passivierungsschicht, wobei die Passivierungsschicht ein Komposit aus einem biodegradierbaren Polymer und Nanopartikeln aus Tonmineralien enthält, wobei das Tonmineral ein organomodifiziertes Tonmineral ist.

2. Implantat nach Anspruch 1, bei dem das Tonmineral Montmorillonit oder Saponit ist.

3. Implantat nach Anspruch 1, bei dem das biodegradierbare Polymer ausgewählt ist aus der Gruppe umfassend Polylactide, Polyhydroxyalkanoate, Polycaprolactone, aliphatische Polyester, aromatische Copolyester und Polyesteramide.

4. Implantat nach Anspruch 3, bei dem das biodegradierbare Polymer ein Polyhydroxyalkonat ist.

5. Implantat nach einem der vorhergehenden Ansprüche, wobei das Implantat ein Stent ist.

6. Implantat nach einem der vorhergehenden Ansprüche, wobei die Passivierungsschicht Hilfsstoffe, vorzugsweise Wirkstoff, besonders bevorzugt pharmakologischen Wirkstoff enthält.

7. Implantat nach Anspruch 6, wobei der pharmakologische Wirkstoff ausgewählt ist aus der Gruppe umfassend Limus-Verbindungen, bevorzugt Sirolimus (Rapamycin), und Cytostatika, vorzugsweise Paclitaxel.

## Claims

1. An implant comprising a main body made of magnesium or a biocorrodible magnesium alloy and a corrosion-inhibiting passivation layer covering the main body, wherein the passivation layer contains a composite of a biodegradable polymer and nanoparticles of clay minerals, wherein the clay mineral is an organo-modified clay mineral.

2. The implant as claimed in claim 1, wherein the clay mineral is montmorillonite or saponite.

3. The implant as claimed in claim 1, wherein the biodegradable polymer is selected from the group comprising polylactides, polyhydroxyalkanoates, polycaprolactones, aliphatic polyesters, aromatic copolyesters and polyester amides.

4. The implant as claimed in claim 3, wherein the biodegradable polymer is a polyhydroxyalkanoate.

5. The implant as claimed in one of the preceding claims, wherein the implant is a stent.

6. The implant as claimed in one of the preceding claims, wherein the passivation layer contains additives, preferably active ingredient, particularly preferably pharmacological active ingredient.

7. The implant as claimed in claim 6, wherein the pharmacological active ingredient is selected from the group comprising limus compounds, preferably sirolimus (rapamycin), and cytostatics, preferably paclitaxel.

## Revendications

1. Implant doté d'un corps de base en magnésium ou dans un alliage de magnésium biocorrodable et d'une couche de passivation inhibant la corrosion recouvrant le corps de base, où la couche de passivation contient un composite à base d'un polymère biodégradable et des nanoparticules à base de minéraux argileux, où le minéral argileux est un minéral argileux modifié par un produit organique.

2. Implant selon la revendication 1, chez lequel le minéral argileux est de la montmorillonite ou de la saponite.

3. Implant selon la revendication 1, chez lequel le polymère biodégradable est choisi dans le groupe comprenant des polylactides, des polyhydroxy alcanoates, des polycaprolactones, des polyesters aliphatiques, des copolyesters aromatiques et des polyester amides.

4. Implant selon la revendication 3, chez lequel le polymère biodégradable est un polyhydroxy alcanoate.

5. Implant selon l'une des revendications précédentes, où l'implant est un stent.

6. Implant selon l'une des revendications précédentes, où la couche de passivation contient des produits auxiliaires, de préférence une matière active, de manière particulièrement préférée, une matière active pharmacologique.

7. Implant selon la revendication 6, dans lequel la matière active pharmacologique est choisie dans le groupe comprenant des composés de la famille des limus, de préférence, du sirolimus (Rapamycine), et des cytostatiques, de préférence, du Paclitaxel.
